# EUROPEAN PATENT APPLICATION

(11) **EP 4 703 481 A1**
(43) Date of publication of application: **04.03.2026**
(21) Application number: 24208042.2
(22) Date of filing: 22.10.2024
(51) Int. Cl.: C12Q 1/6883, C12Q 1/689

(54) **METHOD FOR DETECTING MYCOBACTERIUM SPECIES IN FISH**

(30) Priority: 30.08.2024 EP 24197750
(71) Applicant: Tsiamis, Georgios, 30131 Agrinio (GR); Stathopoulou, Panagiota, 30027 Agrinio (GR); University of Patras, 26504 Rion, Patra (GR)
(72) Inventor: TSIAMIS, Georgios, 30131 Agrinio (GR); STATHOPOULOU, Panagiota, 30027 Agrinio (GR)
(74) Representative: Kosti, Vasiliki

(57) **Abstract**

The present disclosure relates to a novel and rapid method for detecting and identifying *Mycobacterium* species in fish, particularly in sea bass and meagre. The invention also relates to novel DNA primers for use in methods of *Mycobacterium* species detection and kits for use in detecting and identifying said *Mycobacterium* species.

## Description

### FIELD OF THE INVENTION

The present invention relates to a novel and rapid method for detecting and identifying species in fish, particularly in sea bass and meagre. The invention also relates to novel DNA primers for use in methods of *Mycobacterium* species detection and kits for use in detecting and identifying said Mycobacterium species.

### BACKGROUND

Aquaculture is considered as one of the fastest emerging production activities worldwide in the food industry (Dadar et al. 2017). However, one of the most relevant threats in fish farming is the economic loss, due to outbreaks of infectious diseases causing high mortality rates. About 55% of fish infections are caused by bacteria (Dhar et al. 2014), because unlike the terrestrial environment, the aquatic environment favours the development of these microbes regardless of the host. Disease outbreaks in aquaculture are favoured by stocking intensification, which is a strategy to make the production activity more profitable.

Diseases caused by bacteria in fish can be classified into two types, non-granulomatous and granulomatous (Perez Arellano & del Pozo 2013). These last ones pose a great threat to the aquaculture industry, as chronic and necrotizing granulomas can be caused in fish potentially, leading to extensive chronic multifocal granulomatous response, including multiple lesions throughout the affected organs, which severely compromise the immune response of fish and ultimately its survival (Birkbeck et al. 2011).

Mycobacteria, one of the bacterial groups associated with granulomatosis, belong to the family Mycobacteriaceae of the order Actinomycetales. These aerobic, non-motile pleomorphic bacilli are Gram-positive bacteria, despite their mycolic cell wall only fixing the Gram stain poorly, and are usually stained using the Zeihl-Neelsen procedure. The first report of a *Mycobacterium* sp. in fish followed its isolation from granulomatous lesions in common carp (*Cyprinus carpio*) in 1897. Since then, multiple other bacterial species have been described in association with very similar diseases known as mycobacteriosis or "fish tuberculosis".

Mycobacteriosis affects nearly 200 freshwater and saltwater fish species. For example, traditionally, *Mycobacterium marinum, Mycobacterium fortuitum* and *Mycobacterium chelonae* have long been considered as the main etiological agents of fish tuberculosis, while nowadays approximately twenty *Mycobacterium* species are correlated with similar disease symptoms, such as strains of *Mycobacterium pseudoshottsii* and *Mycobacterium hippocampi* isolated from meagre and European sea bass respectively. Given the high incidence of this disease, its economic and public health consequences, there is a real need for rapid and accurate tests, which would allow both the detection and the identification of such infection causing fish mycobacteria.

### SUMMARY

It is a subject of the present invention to provide a novel and rapid method for detecting and identifying *Mycobacterium* species in fish, particularly in sea bass and meagre. It is also subject of the disclosure to provide novel DNA primers for use in methods of *Mycobacterium* species detection and kits for use in detecting and identifying said *Mycobacterium* species.

Based on the disclosure provided herein, those skilled in the art will recognize, or be able to ascertain using no more than routine experimentation, many equivalents to the specific embodiments of the invention described herein. Such equivalents are intended to be encompassed by the following numbered embodiments (E).

E1. A pair of DNA primers for detecting and/or distinguishing a *Mycobacterium* species in a sample isolated by a fish or a marine animal wherein
i. the forward primer of said pair of DNA primers comprises or consists of the sequence of SEQ ID NO: 1 and the reverse primer of said pair of DNA primers comprises or consists of the sequence of SEQ ID NO: 2, and wherein said pair of primers is suitable for detecting and/or distinguishing the presence of *Mycobacterium pseudoshottsii* strain AR and/or *Mycobacterium hippocampi* strain DL in a sample; or
ii. The forward primer of said pair of DNA primers comprises or consists of the sequence of SEQ ID NO: 3 and the reverse primer of said pair of DNA primers comprises or consists of the sequence of SEQ ID NO: 4, and wherein said pair of primers is suitable for detecting and/ or distinguishing the presence of *Mycobacterium marinum* and/or *Mycobacterium pseudoshottsii* strain AR and/or *Mycobacterium hippocampi* strain DL in sample.

E2. The pair of DNA primers of E1 for detecting and/or distinguishing *Mycobacterium* species in a sample isolated by a fish or a marine animal, wherein said detecting and/or distinguishing of Mycobacterium species in a sample is performed by a polymerase chain reaction.

E3. The pair of DNA primers of E2 for detecting and/ or distinguishing *Mycobacterium* species in a sample isolated by a fish or a marine animal, wherein
i. after completion of said polymerase chain reaction using forward primer having the sequence of SEQ ID NO: 1 and reverse primer having the sequence of SEQ ID NO: 2, the production of a DNA fragment having a size of 380 bp indicates the presence of *Mycobacterium hippocampi,* and/or the production of a DNA fragment having a size of 269 bp indicates the presence of *Mycobacterium pseudoshottsii;* or
ii. after completion of said polymerase chain reaction using forward primer having the sequence of SEQ ID NO: 3 and reverse primer having the sequence of SEQ ID NO: 4, the production of a DNA fragment having a size of 904 bp indicates the presence of *Mycobacterium marinum,* and/or the production of a DNA fragment having a size of 831 bp indicates the presence of *Mycobacterium hippocampi,* and/or the production of a DNA fragment having a size of 538 bp indicates the presence of *Mycobacterium pseudoshottsii.*

E4. A composition comprising a primer pair selected from the group consisting of SEQ ID NOs: 1 and 2 and SEQ ID NOs: 3 and 4.

E5. The composition of E4, wherein said composition is a reaction mixture.

E6. The composition of E5, wherein said reaction mixture comprises a sample.

E7. The composition of E6, wherein said sample comprises a *Mycobacterium marinum* or *Mycobacterium hippocampi* or *Mycobacterium pseudoshottsii* target nucleic acid.

E8. A method of detecting and/ or distinguishing the presence of a *Mycobacterium* strain in a sample isolated by a fish or marine animal, characterised in that a pair of DNA primers according to any one of E1 to E3 is used in order to amplify a substrate DNA comprised in said sample.

E9. The method of E8, wherein said method is polymerase chain reaction.

E10. The method of E9, wherein
i. after completion of said polymerase chain reaction using forward primer having the sequence of SEQ ID NO: 1 and reverse primer having the sequence of SEQ ID NO: 2, the production of a DNA fragment having a size of 380 bp indicates the presence of *Mycobacterium hippocampi,* and/or the production of a DNA fragment having a size of 269 bp indicates the presence of *Mycobacterium pseudoshottsii;* or
ii. after completion of said polymerase chain reaction using forward primer having the sequence of SEQ ID NO: 3 and reverse primer having the sequence of SEQ ID NO: 4, the production of a DNA fragment having a size of 904 bp indicates the presence of *Mycobacterium marinum,* and/or the production of a DNA fragment having a size of 831 bp indicates the presence of *Mycobacterium hippocampi,* and/or the production of a DNA fragment having a size of 538 bp indicates the presence of *Mycobacterium pseudoshottsii.*

E11. A kit for detecting or distinguishing the presence of a *Mycobacterium* in a sample isolated by a fish or marine animal, wherein said kit comprises a pair of DNA primers according to any one of E1 to E3 or a composition according to claims E4 to E7.

### BRIEF DESCRIPTION OF THE DRAWINGS

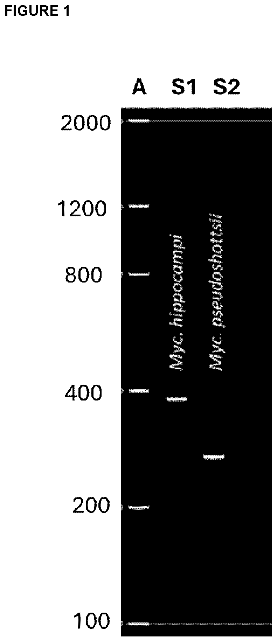

**Figure 1****:** Gel electrophoresis of amplified DNA products from two bacterial samples isolated by a *Argyrosomus regius* fish "S2" and *Dicentrarchus labrax* fish "S1" (agarose gel 1.5% w/v). PCR products were electrophoresed on a 1.5% (w/v) of agarose gel in TAE buffer (1X) (40 mM Tris-acetate, 1 mM EDTA). Amplification products were visualized in Bio-Rad's Gel Doc^{™} XR+ system.
As it can be seen, the PCR product from sample S1 has a size of 380 bp identifying the presence of *Mycobacterium hippocampi,* while PCR product from sample S2 has a size of 269 bp identifying the presence of *Mycobacterium pseudoshottsii.* Lane A is a DNA ladder control with DNA fragments of known size.

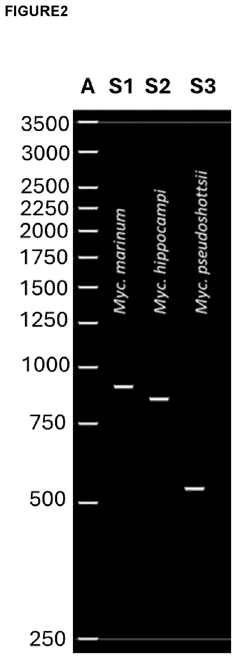

**Figure 2****:** Gel electrophoresis of amplified DNA products from three bacterial samples isolated by a *Argyrosomus regius* fish "S3" and *Dicentrarchus labrax* fish "S1", "S2" (agarose gel 1.5% w/v). PCR products were electrophoresed on a 1.5% (w/v) of agarose gel in TAE buffer (1X) (40 mM Tris-acetate, 1 mM EDTA). Amplification products were visualized in Bio-Rad's Gel Doc^{™} XR+ system. PCR product from sample S1 has a size of 904 bp identifying the presence of *Mycobacterium marinum,* PCR product from sample S2 has a size of 831 bp identifying the presence of *Mycobacterium hippocampi,* while PCR product from sample S3 has a size of 538 bp identifying the presence of *Mycobacterium pseudoshottsii.* Lane A is a DNA ladder control with DNA fragments of known size.

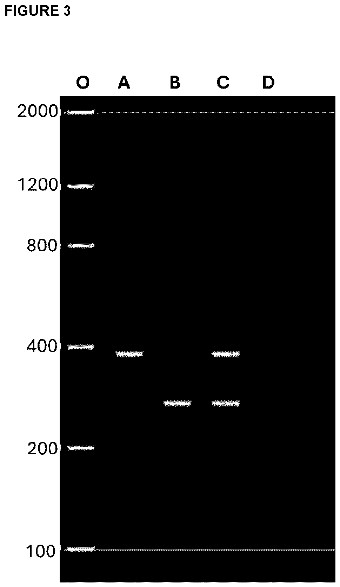

**Figure 3****:** Gel electrophoresis of amplified DNA products from four tissue samples, with two samples isolated from *Argyrosomus regius* fish tissue (B and D) and two samples isolated from *Dicentrarchus labrax* fish tissue (A and C). The PCR product from sample A, measuring 380 bp, indicates the presence of *Mycobacterium hippocampi.* Sample B's PCR product, measuring 269 bp, identifies the presence of *Mycobacterium pseudoshottsii.* Sample C shows two bands: one at 380 bp, indicating *Mycobacterium hippocampi,* and another at 269 bp, indicating the co-presence of *Mycobacterium pseudoshottsii.* No amplification was observed for sample D, suggesting the absence of both *Mycobacterium pseudoshottsii* strain AR and *Mycobacterium hippocampi* strain DL. Lane O is a DNA ladder control with DNA fragments of known size.

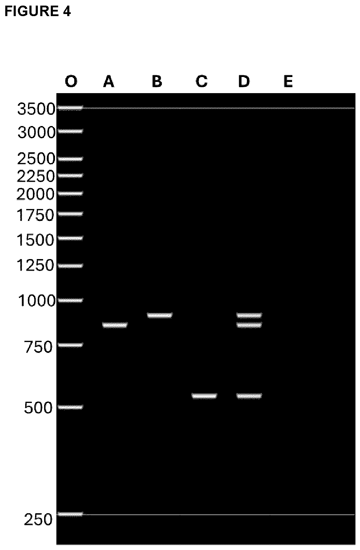

**Figure 4****:** Gel electrophoresis of amplified DNA products from five tissue samples, with two samples from *Argyrosomus regius* fish (C and E) and three from *Dicentrarchus labrax* fish (A, B, and D). The PCR product from sample A, measuring 831 bp, indicates the presence of *Mycobacterium hippocampi.* Sample B's PCR product, at 904 bp, identifies *Mycobacterium marinum.* Sample C's PCR product, sized at 538 bp, reveals the presence of *Mycobacterium pseudoshottsii.* Sample D shows three bands: one at 831 bp, indicating *Mycobacterium hippocampi;* another at 904 bp, indicating the co-presence of *Mycobacterium marinum;* and a third at 538 bp, indicating the co-presence of *Mycobacterium pseudoshottsii.* For sample E, no amplification was observed, suggesting the absence of *Mycobacterium pseudoshottsii* strain AR, *Mycobacterium marinum,* and *Mycobacterium hippocampi* strain DL. Lane O is a DNA ladder control with DNA fragments of known size.

### DETAILED DESCRIPTION

The present disclosure relates to to a novel and rapid method for detecting and identifying *Mycobacterium* species in fish, particularly in sea bass and meagre. The invention also relates to novel DNA primers for use in methods of *Mycobacterium* species detection and kits for use in detecting and identifying said *Mycobacterium* species.

Applicants and inventors have previously isolated a *Mycobacterium pseudoshottsii* strain AR, isolated from infected meagre (*Argyrosomus regius*) raised on farms in Greece (Stathopoulou et al; 2020; https://doi.org/10.1128/MRA.01099-20 and a *Mycobacterium hippocampi* strain DL, isolated from cultured European Sea Bass (*Dicentrarchus labrax*) (Stathopoulou et al; 2020; https://doi.orq/10.1128/MRA.00816-20). Both meagre and European sea bass are considered to be among the best potential candidates for large-scale fish farming in the Mediterranean sea. Although global meagre and sea bass production is reported to have increased rapidly, one of the most important bottlenecks remains systemic granulomatosis, mainly associated to mycobacteriosis. Therefore, there is an emerging need for timely and precise detection and identification of such bacterial strains, allowing for early control of disease and limiting of infection expansion.

The inventors of the current disclosure designed a novel set of DNA primers for use in methods of molecular detection of *Mycobacterium pseudoshottsii* strain AR and *Mycobacterium hippocampi* strain DL in samples of infected fish. The specifically designed set of primers not only allows the detection of said mycobacterium strains but also allows for accurate species determination and distinguish between the presence of said species and *Mycobacterium marinum,* a broadly widespread fish pathogen causing similar disease symptoms.

Therefore, in one aspect the present disclosure refers to a pair of DNA primers for detecting and/or distinguishing a *Mycobacterium* species in a sample isolated by a fish or a marine animal.

In one embodiment, the disclosure refers to a pair of DNA primers for detecting and/or distinguishing *Mycobacterium pseudoshottsii* strain AR from *Mycobacterium hippocampi* strain DL and/or *Mycobacterium marinum* in a sample isolated by a fish or a marine animal.

In one embodiment the forward primer of said pair of DNA primers comprises or consists of the sequence of SEQ ID NO: 1 and the reverse primer of said pair of DNA primers comprises or consists of the sequence of SEQ ID NO: 2.

In one embodiment the forward primer of said pair of DNA primers comprises or consists of the sequence of SEQ ID NO: 3 and the reverse primer of said pair of DNA primers comprises or consists of the sequence of SEQ ID NO: 4.

In one embodiment,
i. the forward primer of said pair of DNA primers comprises or consists of the sequence of SEQ ID NO: 1 and the reverse primer of said pair of DNA primers comprises or consists of the sequence of SEQ ID NO: 2, and wherein said pair of primers is suitable for detecting and/or distinguishing the presence of *Mycobacterium pseudoshottsii* strain AR and *Mycobacterium hippocampi* strain DL; or
ii. the forward primer of said pair of DNA primers comprises or consists of the sequence of SEQ ID NO: 3 and the reverse primer of said pair of DNA primers comprises or consists of the sequence of SEQ ID NO: 4, and wherein said pair of primers is suitable for detecting and/ or distinguishing the presence of *Mycobacterium marinum, Mycobacterium pseudoshottsii* strain AR and *Mycobacterium hippocampi* strain DL.

In one embodiment, said detecting and/or distinguishing of *Mycobacterium pseudoshottsii* strain AR from *Mycobacterium hippocampi* strain DL and/or *Mycobacterium marinum* in a sample isolated by a fish or a marine animal is performed by a polymerase chain reaction.

In one embodiment said pair of DNA primers for detecting and/ or distinguishing *Mycobacterium* species in a sample isolated by a fish or a marine animal is characterised in that
i. after completion of said polymerase chain reaction using forward primer having the sequence of SEQ ID NO: 1 and reverse primer having the sequence of SEQ ID NO: 2, the production of a DNA fragment having a size of 380 bp indicates the presence of *Mycobacterium hippocampi,* and/or the production of a DNA fragment having a size of 269 bp indicates the presence of *Mycobacterium pseudoshottsii;* or
ii. after completion of said polymerase chain reaction using forward primer having the sequence of SEQ ID NO: 3 and reverse primer having the sequence of SEQ ID NO: 4, the production of a DNA fragment having a size of 904 bp indicates the presence of *Mycobacterium marinum,* and/or the production of a DNA fragment having a size of 831 bp indicates the presence of *Mycobacterium hippocampi,* and/or the production of a DNA fragment having a size of 538 bp indicates the presence of *Mycobacterium pseudoshottsii.*

In one embodiment said pair of DNA primers for detecting and/ or distinguishing *Mycobacterium* species in a sample isolated by a fish or a marine animal is characterised in that
iii. after completion of said polymerase chain reaction using forward primer having the sequence of SEQ ID NO: 1 and reverse primer having the sequence of SEQ ID NO: 2, the production of a DNA fragment having the sequence of SEQ ID NO: 5 indicates the presence of *Mycobacterium hippocampi,* and/or the production of a DNA fragment having the sequence of SEQ ID NO:6 indicates the presence of *Mycobacterium pseudoshottsii;* or
iv. after completion of said polymerase chain reaction using forward primer having the sequence of SEQ ID NO: 3 and reverse primer having the sequence of SEQ ID NO: 4, the production of a DNA fragment having the sequence of SEQ ID NO: 7 indicates the presence of *Mycobacterium marinum,* and/or the production of a DNA fragment having the sequence of SEQ ID NO: 8 indicates the presence of *Mycobacterium hippocampi,* and/or the production of a DNA fragment having the sequence of SEQ ID NO: 9 indicates the presence of *Mycobacterium pseudoshottsii.*

In another aspect the disclosure refers to a composition comprising a primer pair selected from the group consisting of SEQ ID NOs: 1 and 2 and SEQ ID NOs: 3 and 4.

In one embodiment said composition comprises a pair of primers wherein the forward primer of said pair comprises the sequence of SEQ ID NO:1 and the reverse primer of said pair comprises the sequence of SEQ ID NO:2.

In one embodiment said composition comprises a pair of primers wherein the forward primer of said pair consists of the sequence of SEQ ID NO:1 and the reverse primer of said pair consists of the sequence of SEQ ID NO:2.

In one embodiment said composition comprises a pair of primers wherein the forward primer of said pair comprises the sequence of SEQ ID NO:3 and the reverse primer of said pair comprises the sequence of SEQ ID NO:4.

In one embodiment said composition comprises a pair of primers wherein the forward primer of said pair consists of the sequence of SEQ ID NO:3 and the reverse primer of said pair consists of the sequence of SEQ ID NO:4.

In one embodiment, said composition is for detecting and/or distinguishing *Mycobacterium pseudoshottsii* strain AR from *Mycobacterium hippocampi* strain DL and/or *Mycobacterium marinum* in a sample isolated by a fish or a marine animal.

In one embodiment said composition is a reaction mixture.

In one embodiment said composition is a polymerase reaction mixture

In one embodiment said reaction mixture additionally comprises a sample.

In one embodiment said sample comprises a *Mycobacterium marinum* and/or *Mycobacterium hippocampi* and/or *Mycobacterium pseudoshottsii* target nucleic acid, suitably DNA.

In one embodiment said sample comprises a *Mycobacterium marinum* or *Mycobacterium hippocampi* and/or *Mycobacterium pseudoshottsii* target nucleic acid, suitably DNA.

In a further aspect the present disclosure relates to a method of detecting and/ or distinguishing the presence of a *Mycobacterium* strain in a sample isolated by a fish or marine animal, characterised in that a pair of DNA primers according to the disclosure is used in order to amplify a substrate DNA comprised in said sample.

In one embodiment said method is a polymerase chain reaction.

In one embodiment, said method is characterised in that after completion of said polymerase chain reaction using forward primer having the sequence of SEQ ID NO: 1 and reverse primer having the sequence of SEQ ID NO: 2, the production of a DNA fragment having a size of 380 bp indicates the presence of *Mycobacterium hippocampi,* and/or the production of a DNA fragment having a size of 269 bp indicates the presence of *Mycobacterium pseudoshottsii.*

In one embodiment, said method is characterised in that after completion of said polymerase chain reaction using forward primer having the sequence of SEQ ID NO: 1 and reverse primer having the sequence of SEQ ID NO: 2, the production of a DNA fragment having the sequence of SEQ ID NO:5 indicates the presence of *Mycobacterium hippocampi,* and/or the production of a DNA fragment having the sequence of SEQ ID NO:6 indicates the presence of *Mycobacterium pseudoshottsii.*

In one embodiment, said method is characterised in that after completion of said polymerase chain reaction using forward primer having the sequence of SEQ ID NO: 3 and reverse primer having the sequence of SEQ ID NO: 4, the production of a DNA fragment having a size of 904 bp indicates the presence of *Mycobacterium marinum,* and/or the production of a DNA fragment having a size of 831 bp indicates the presence of *Mycobacterium hippocampi,* and/or the production of a DNA fragment having a size of 538 bp indicates the presence of *Mycobacterium pseudoshottsii.*

In one embodiment, said method is characterised in that after completion of said polymerase chain reaction using forward primer having the sequence of SEQ ID NO: 3 and reverse primer having the sequence of SEQ ID NO: 4, the production of a DNA fragment having the sequence of SEQ ID NO: 7 indicates the presence of *Mycobacterium marinum,* and/or the production of a DNA fragment having the sequence of SEQ ID NO:8 indicates the presence of *Mycobacterium hippocampi,* and/or the production of a DNA fragment having the sequence of SEQ ID NO:9 indicates the presence of *Mycobacterium pseudoshottsii.*

In one embodiment, said method is characterised in that
i. after completion of said polymerase chain reaction using forward primer having the sequence of SEQ ID NO: 1 and reverse primer having the sequence of SEQ ID NO: 2, the production of a DNA fragment having a size of 380 bp indicates the presence of *Mycobacterium hippocampi,* and/or the production of a DNA fragment having a size of 269 bp indicates the presence of *Mycobacterium pseudoshottsii;* or
ii. after completion of said polymerase chain reaction using forward primer having the sequence of SEQ ID NO: 3 and reverse primer having the sequence of SEQ ID NO: 4, the production of a DNA fragment having a size of 904 bp indicates the presence of *Mycobacterium marinum,* and/or the production of a DNA fragment having a size of 831 bp indicates the presence of *Mycobacterium hippocampi,* and/or the production of a DNA fragment having a size of 538 bp indicates the presence of *Mycobacterium pseudoshottsii.*

In one embodiment, said method is characterised in that
iii. after completion of said polymerase chain reaction using forward primer having the sequence of SEQ ID NO: 1 and reverse primer having the sequence of SEQ ID NO: 2, the production of a DNA fragment having the sequence of SEQ ID NO:5 indicates the presence of *Mycobacterium hippocampi,* and/or the production of a DNA fragment having the sequence of SEQ ID NO: 6 indicates the presence of *Mycobacterium pseudoshottsii;* or
iv. after completion of said polymerase chain reaction using forward primer having the sequence of SEQ ID NO: 3 and reverse primer having the sequence of SEQ ID NO: 4, the production of a DNA fragment having the sequence of SEQ ID NO:7 indicates the presence of *Mycobacterium marinum,* and/or the production of a DNA fragment having the sequence of SEQ ID NO: 8 indicates the presence of *Mycobacterium hippocampi,* and/or the production of a DNA fragment having the sequence of SEQ ID NO 9: indicates the presence of *Mycobacterium pseudoshottsii.*

In another aspect the current disclosure refers to a kit for detecting and/or distinguishing the presence of a *Mycobacterium* strain in a sample isolated by a fish or marine animal, wherein said kit comprises a pair of DNA primers wherein the forward primer of said pair comprises or consists of the sequence of SEQ ID NO:1 and the reverse primer of said pair comprises or consists of the sequence of SEQ ID NO:2.

In one embodiment said kit comprises a pair of DNA primers wherein the forward primer of said pair comprises or consists of the sequence of SEQ ID NO:3 and the reverse primer of said pair comprises or consists of the sequence of SEQ ID NO:4.

In one embodiment said kit comprises a composition of the disclosure.

### Definitions

Unless otherwise defined, scientific and technical terms used herein have the meanings that are commonly understood by those of ordinary skill in the art. In the event of any latent ambiguity, definitions provided herein take precedent over any dictionary or extrinsic definition.

The term "about" or "approximately" means the mentioned value +/-10%, for example about 10 shall mean 9 to 11.

The term "primer or "DNA primer" in the context of the present disclosure is a short, single-stranded segments of DNA or that is designed to be complementary to the beginning or end of a target sequence that will be amplified. In a Polymerase Chain Reaction (PCR), it is the primers that dictate exactly what sequence of DNA gets copied. A standard PCR uses two primers, often called the "forward" and "reverse" primers. The forward and reverse primers are oriented on opposite strands of the DNA. During a PCR run, the primers bind to the DNA, bookending the sequence intended for amplification. DNA polymerase then copies the part of the target sequence that falls between the primers, selectively amplifying the sequence of interest. PCR primers are the main determinants of PCR specificity. In order to amplify a specific DNA sequence, the sequence of the primer annealing sites on the target DNA must be known. Further, in order to limit the chance of non-specific primer binding (where one or more bases are not complementary to the target sequence), or the chance of the primers binding to a similar sequence elsewhere on the target DNA, primers are usually designed to be in the order of 18-22 nucleotides in length

The terms "pair of primers", "pair of DNA primers", "primer pair", "primers pair", "primers set", in the context of the present disclosure, are used interchangeably.

A "Polymrase Chain Reaction" refers to a nucleic acid amplification testing procedure. The majority of PCR methods rely on thermal cycling. Thermal cycling exposes reagents to repeated cycles of heating and cooling to permit different temperature-dependent reactions-specifically, DNA melting and enzyme-driven DNA replication. PCR employs two main reagents-primers (which are short single strand DNA fragments known as oligonucleotides that are a complementary sequence to the target DNA region) and a thermostable DNA polymerase. In the first step of PCR, the two strands of the DNA double helix are physically separated at a high temperature in a process called nucleic acid denaturation. In the second step, the temperature is lowered and the primers bind to the complementary sequences of DNA. The two DNA strands then become templates for DNA polymerase to enzymatically assemble a new DNA strand from free nucleotides, the building blocks of DNA. As PCR progresses, the DNA generated is itself used as a template for replication, setting in motion a chain reaction in which the original DNA template is exponentially amplified. Almost all PCR applications employ a heat-stable DNA polymerase, such as Taq polymerase, an enzyme originally isolated from the thermophilic bacterium Thermus aquaticus.

In general "Nucleic acid amplification", such as in a polymerase chain reaction, require reagents. "Nucleic acid amplification reagents" include but are not limited to, an enzyme having at least polymerase activity, enzyme cofactors such as magnesium or manganese; salts; and deoxynucleotide triphosphates (dNTPs) such as for example deoxyadenine triphosphate, deoxyguanine triphosphate, deoxycytosine triphosphate and deoxythymine triphosphate.

In the context of the current disclosure a "method of distinguishing a *Mycobacterium* speceis" shall refer to a method which allows for distinguishing the presence of one *Mycobacterium* species in a sample from an other *Mycobacterium* species.

Amplification conditions are conditions that generally promote annealing and extension of one or more nucleic acid sequences.

Unless otherwise required by context, singular terms shall include pluralities and plural terms shall include the singular. The use of "or" means "and/or" unless stated otherwise.

The use of "comprise," "comprises," "comprising" "include," "includes," and "including" are interchangeable and not intended to be limiting. Furthermore, where the description of one or more embodiments uses the term "comprising," those skilled in the art would understand that in some specific instances, the embodiment or embodiments can be alternatively described using language "consisting essentially of" and/or "consisting of."

A "target sequence" or "target nucleic acid sequence" as used herein means a nucleic acid sequence of *Mycobacterium marinum* or *Mycobacterium hippocampi* or *Mycobacterium pseudoshottsii* or complement thereof, that is amplified, detected, or both amplified and detected using one or more of the primers herein provided. Additionally, while the term target sequence sometimes refers to a double stranded nucleic acid sequence, those skilled in the art will recognize that the target sequence can also be single stranded. In cases where the target is double stranded, primer sequences preferably amplify both strands of the target sequence. A target sequence may be selected that is more or less specific for a particular organism. For example, the target sequence may be specific to an entire genus, to more than one genus, to a species or subspecies, serogroup, auxotype, serotype, strain, isolate or other subset of organisms.

The contents of the articles, patents, and patent applications, and all other documents and electronically available information mentioned or cited herein, are hereby incorporated by reference in their entirety to the same extent as if each individual publication was specifically and individually indicated to be incorporated by reference. Applicants reserve the right to physically incorporate into this application any and all materials and information from any such articles, patents, patent applications, or other physical and electronic documents.

While the present invention has been described with reference to the specific embodiments thereof, it should be understood by those skilled in the art that various changes may be made and equivalents may be substituted without departing from the true spirit and scope of the invention using this disclosure as a guide. Having now described certain embodiments in detail, the same will be more clearly understood by reference to the following examples, which are included for purposes of illustration only and are not intended to be limiting.

### EXAMPLES

### General techniques

The practice of the present invention employs, unless otherwise indicated, conventional techniques of molecular biology, microbiology, animal cell culture and biochemistry which are within the skill of the art. Such techniques are explained fully in the literature. See, for example, "Molecular Cloning: A Laboratory Manual", Second Edition (Sambrook, Fritsch & Maniatis, 1989); "Animal Cell Culture" (R.I. Freshney, ed., 1987); "Current Protocols in Molecular Biology" (F.M. Ausubel et al., eds., 1987); "PCR:Methods and Protocols" ( L. Domingues, Springer Protocols, 2023)

### Example 1: Design of DNA primers for specific molecular identification of Mycobacterium pseudoshottsii strain AR and Mycobacterium hippocampi strain DL.

In order to deisgn primers for the detection of a single gene capable of simultaneously detecting and distinguishing mycobacterial isolates, the whole genome sequences of *Mycobacterium pseudoshottsii* strain AR and *Mycobacterium hippocampi* strain DL were aligned so that common genes of different length are identified. The whole genomic DNA of of *M*. *pseudoshottsii* has been described and sequenced previously (https://doi.orq/10.1128/MRA.01099-20) and is deposited at DDBJ/ENA/GenBank under the accession number JABXJI000000000.1. Similarly the whole genomic DNA of of *M*. *hippocampi* has been described and sequenced previously (https://doi.org/10.1128/MRA.00816-2) and is deposited at DDBJ/ENA/GenBank under the accession number JABFYL000000000.1. For the genomic sequence alignment (multiple alignments were performed) the MUSCLE alignment tool was used ("Muscle Sequence Alignement MSA", https://www.ebi.ac.uk/jdispatcher/msa/muscle)

The whole genomic DNA alignment led to the identification of a coding region of a glycosyltransferase (Bifunctional beta-1,5/1,6-galactofuranosyltransferase GlfT2, involved in cell wall polymerization) both from the *Mycobacterium* strain isolated from the meagre (Accession: NZ_JABXJI010000031 Region: 26043-27941; Locus tag: HU829_RS02145) and from the *Mycobacterium* strain isolated from the sea bass (NZ_JABFYL010000035 Region: 113089-115152; Locus tag: HLY00_RS15715). The selected conserved coding sequence were the basis for the primers design using the primer designing tool "Primer-BLAST" (hftps://www.ncbi.nlm.nih.gov/tools/primer-blas/), and the following primers were selected: forward primer Myc2_185F (SEQ ID NO 1: 5' -AATCCGAGGTCTCCTTCGC-3') and reverse primer Myc2_564R (SEQ ID NO 2: 5'-GTCGGTGGTGATGTCGAACC- 3'). DNA amplification of the genomic DNA of *Mycobacterium pseudoshottsii* strain AR and *Mycobacterium hippocampi* strain DL, using standard techniques known in the prior art (i.e. Polymerase Chain Reaction) and the selected DNA primers mentioned above, leads to DNA products with a size of 269 bp and 380 bp respectively (see Figure 1). In brief, for the DNA amplification one cycle at 95 0C for 2 min was performed, followed by 35 cycles at 95 0C for 30 seconds (denaturation), at 50 0C for 30 seconds (annealing), at 72 ⁰C for 50 seconds (extending), and one cycle at 72 ⁰C for 2 min. The DNA amplification was performed in 25 µL reaction mixtures containing 2.5 µL KAPA Taq buffer (KAPA BioSystems, Wilmington,USA) 10×, 0.25 µL dNTPs (25 mM), 0.25 µL of KAPA Taq DNA polymerase, 0.5 µL of the forward primer (25 µM), 0.5 µL of the reverse primer (25 µM), 1 µL of template DNA solution, and was finalized with 20 µL sterile deionized water. The amplified PCR products were then run in an agarose gel electrophoresis in order to identify and the size and thus the origin of the amplified DNA fragment.

The DNA sequences of the amplification products are as follows (5' to 3'):
**1. DNA sequence (SEQ ID NO:5) of amplification product with a size of 269 bp from *Mycobacterium pseudoshottsii* strain AR,** 
AATCCGAGGTCTCCTTCGCCACCTACTTCAATGCGTTTCCGGCCAGCTACTGGCGACGCTGG
TCGATCTGTAAGTCCGTGGTGCTGCGCGTCGAGTTGTCCGGTGCCGGGCGCGTCGACGTCT
ACCGGACCAAGGCCACCGGAGCGCGCATCTTCGTCGAAGGGCGCGCATTTGCCGGTGATGA
GGACAAGCCGGCCGTGCTCGAGATCGAGGTGGCGCTGACGCCGTTTGAAGACGGCGGCTG
GATCTGGTTCGACATCACCACCGAC
**2. DNA sequence (SEQ ID NO:6) of amplification product with a size of 380 bp from *Mycobacterium hippocampi* strain DL**

AATCCGAGGTCTCCTTCGCGACCTACTTCAACGCCTTCCCGGCCAGTTACTGGCGCCGCTGG
TCGACGCTGGAATCAGTGGTGTTGCGTGTCGAACTCACCGGCAGCGCGCGCGTCGACGTCT
ACCGGTCCAAGGCCACCGGGTCCCGGATAACCGTCGGCGGGGCGCCGATCTCCAGCGAAAA
TGTCGGCGCGAGCGCCTCGGGATCTGATGGCGGCGCAAGCGCCTCGGGATCTGATGTCGGC
GCAAGCGCCTCGGGATCTGATGTCGGCGCAAGCGCCTCGGGATCTGATGTCGGCGCGAGCG
CCTCCGTGGAGTTCGAGGTCGACCTCACCCCGTTCGAGGACGGTGGCTGGATCTGGTTCGA
CATCACCACCGAC

As it can be seen in Figure 1 which illustrates, a gel electrophoresis of the DNA apmplification rpoducts, the PCR product from sample S1 has a size of 380 bp identifying the presence of Mycobacterium hippocampi, while PCR product from sample S2 has a size of 269 bp identifying the presence of Mycobacterium pseudoshottsii. Lane A is a DNA ladder control with DNA fragments of known size.

### Example 2: Design of DNA primers for molecular identification of Mycobacterium pseudoshottsii strain AR, Mycobacterium hippocampi strain DL and Mycobacterium marinum.

In order to deisgn primers for the specific detection *Mycobacterium pseudoshottsii* strain AR, *Mycobacterium hippocampi* strain DL and *Mycobacterium marinum* respectively, the whole genomic DNA of the three bacterial species were aligned so that common genes of different length are identified, in a similar process with that descibed in Example 1. For the sequence alignment, the whole genomic DNA of *Mycobacterium marinum* (strain DSM 43518, NCBI Reference Sequence: NZ_PEDA00000000.1; strain DSM 44344 NCBI, Reference Sequence: NZ_PEDC00000000.1) was used along with the genomic DNA used in Example 1.

The whole genomic DNA alignment led to the identification of a coding region of prolipoprotein diacylglyceryl transferase present in the genomes of the four mycobacterial strains *(Mycobacterium pseudoshottsii* strain AR, Accession: CP155055, REGION: 2651814..265235, *Mycobacterium hippocampi* strain DL, Accession: CP155796 REGION: 1212182..1213012 and *Mycobacterium marinum strain* DSM 43518 DSM43518_contig000025 REGION: 88247-89364 and *Mycobacterium marinum* strain DSM 44344 DSM44344_contig000019 REGION: 88252-89369). The selected conserved coding sequence were the basis for the primers design using the primer designing tool "Primer-BLAST" (https://www.ncbi.nlm.nih.gov/tools/primer-blast/), and the following primers were selected: forward primer Mmar_98F (SEQ ID NO 4: 5' GCGGTCGACGGTGAGGTC 3') and reverse primer Mpsd1063 mar1001 R (SEQ ID NO 5: 5' GGCTCTGCTCCAGCTCACTT 3').

DNA amplification of the genomic DNA of *Mycobacterium pseudoshottsii* strain AR, *Mycobacterium hippocampi* strain DL and *Mycobacterium marinum,* using standard techniques known in the prior art (i.e Polymerase Chain Reaction) and the selected primers leads to DNA products with a size of 538 bp, 831 bp and 904 bp respectively (see also Figure 2). In brief, for the DNA amplification one cycle at 95 ⁰C for 2 min was performed, followed by 35 cycles at 95 ⁰C for 30 seconds (denaturation), at 50 ⁰C for 30 seconds (annealing), at 72 ⁰C for 50 seconds (extending), and one cycle at 72 ⁰C for 2 min. The DNA amplification was performed in 25 µL reaction mixtures containing 2.5 µL KAPA Taq buffer (KAPA BioSystems, Wilmington,USA) 10×, 0.25 µL dNTPs (25 mM), 0.25 µL of KAPA Taq DNA polymerase, 0.5 µL of the forward primer (25 µM), 0.5 µL of the reverse primer (25 µM), 1 µL of template DNA solution, and was finalized with 20 µL sterile deionized water. The amplified PCR products were then run in an agarose gel electrophoresis in order to identify and the size and thus the origin of the amplified DNA fragment

The DNA sequences of the amplification products are as follows (5' to 3'):
**1. DNA seequence (SEQ ID NO: 7) of amplification product with** a **size of 538 bp from *Mycobacterium pseudoshottsii* strain AR** 
GCGGTCGAGGGTGAGGTCGAAGTGGCCGCTGAGGCCGAAGAGGCTGAGGCGGGCGAGGAC
GAGCCAAAAGAGGCAGAAGAGGTCGCTGAGGCCGAGGGGTCTGAGGCCGAGGACAAGACA
GCGCAGGCCATAGATGGTGGCCAAAAGGGGGTTGACGAGGCAGGCGAGGCCGATGCTGAG
CCAGACGACGACTCCACCGCGGCAGAACCCGAGGTCGTGGTCGAGCAAGCAGATATCGAAG
AGATCGAACCCCAAGAGCCCGAAGCGGAGGCGGTGGCGGCCGCTGAAGCGACGGACGCCG
ACGAGGGTGATGGCGAGCCCGCCGAGCCGGCGAGCGACGATCTCGACGCTGTCGCGGCCG
AGGACGCTGACGTTGACGTTGACGCCACGGCAACCGAAGCCGAAGAATCCGGCGATGAGTC
 
GCCCGAGCCCGCCGCAGACTCCGAGGAGCCCGAAGCCGACGAGTCCGCACCGGAGGACTC
GGCCGAGGACACTTCGTCCGGTGAATCCGACACCCAAAGTGAGTTGGAGCAGAGCC
**2. DNA sequence (SEQ ID NO: 8) of amplification product with a size of 831 bp from *Mycobacterium hippocampi* strain DL** 
CATGGGACCCCACCACGCTTCGTTGTCGCGGTACCAGGTGATGGTGTCGCGCAGGCCGGCC
TCGAAGTCGGTGTGCGCCGGCTTCCAGCCCAGCTCGTCACGCAGCGGTGACGGATCGATCG
CGTACCGCAGATCGTGGCCCACCCGGTCGGTGACGTGGTCGAACTCGTCGGTGGGTCGGTC
CATCAGCGTCAGGATCGTGCGCATCACGGACAGATTGTCGTGTTCGCCGTCGGCTCCGATCA
GATACGTGCGCCCGATCTCGCCGTCTGCGAGGATCCGCCACACCGCACTGTTGTGGTCGTC
GACATGGATCCAGTCGCGCACATTGGCACCGCTGCCGTACAGCTTGGGGCGCCGGCCGGTC
AGCACATTGGTGATCTGGCGCGGAATGAACTTCTCGACATGCTGGTAGGGGCCGTAGTTGTT
GGAGCAGTTCGAGATCGTCGCCGCCACCCCGTAGGAGCGCACCCACGCCCGGACCAGCATG
TCGGCCGCCGCCTTGGTCGACGAGTACGGGCTGGACGGGTTGTACGCAGTCGACTCGGTGA
ATCGTCGCGGATCGTCGAGCGTCAGATCGCCGTACACCTCGTCGGTGGACACATGGTGCAAC
CGGATGCCCGCCTCAGAACCTCTGTGGCGCACCGCTTCCAGTACCGAGAACGTACCGAGGA
CATTCGACCGCACGAACGGCTCGGGATCGGCCAGTGCGTTGTCGACGTGGGTCTCCGCGGC
GAAGTGCACCACCGCATCGGACTCGGCGACGAGCTTGCCCACCAGCGGCGCATCGGTGATA
TCACCCTGCACCAACCGGATCTCACCGTCGACCGC
**3. DNA sequence (SEQ ID NO: 9) of amplification product with a size of 904 bp from *Mycobacterium marinum***

GCGGTCGACGGTGAGGTCGAGGCGGCTGAGGCGGGCGAGGTAGAGGTTGCTGAGGCGGTC
GAGGGTGAGGTTGAGGCGGCTGAGGCGGGCGAGGTAGAGGTTGCTGAGGCGGTCGAGGGT
GAGGTTGAGGCGGCTGCTGAGGCTGAGATGCCTGAGCTGGGTGAGGTAGAAGCTGCTGAGG
AGGCCGATGAGGCGGGNGAGGGNGAGGTAGAAGCGGTTGAGGAGGCGGCTGAGGCCGAG
GCACCCGAGGTGGGTGAGGTAGAGGTTGCTGAGGCGGTCGACGGTGAGGTTGAGGAGGCG
GCTGAGCCTGAAGAGGCTGAGGTTGAGGAGGCGGCTGAGCCTGAAGAGGCTGAGGCGGGN
GAGGNAGAGGTTGCTGAGGCGATCGAGGGNGGGGTCGACGAGGCCGCTGAGGCCGAAGAG
GCTGAGGCGGGCGAGGACGAGCCAAAAGAGGCAGCAGAGGTCGCTGAGGCCGAGGGGTCT
GAGGCCGAGGACAAGACAGCGCAGGCCATAGATGGTGGCCAAACGGGGGTTGACGACGCA
GACGAGGCCGATGCTGAGCCAGACGACGACTCCACCGCGGCAGAACCCGAGGTCGTGGTCG
AGCAAGCAGATATCGAAGAGATCGAACCCCAAGAGCCCGAAGCGGAGGCATTGGCGGCCGC
TGAAGCGACGGACGCCGACGAGGGTGATGGCGAGCCCGCCGAGCCGGCGAGCGACGATCT
CGACGCTGTCGCGGCCGAGGACGCTGACGCCACGGCAACCGAAGCCGAAGAATCCGGCGAT
GAGTCGCCCGAGCCCGCCGCAGACTCCGAGGAGCCCGGAGCCGACGAGTCCGCACCGGAG
GACTCAGCCGAGGACACTTCGTCCGGTGAATCCGACACCCAAAGTGAGCTGGAGCAGAGCC

As it can be seen in Figure 2, which illustrates a gel electrophoresis of the DNA amplification products, PCR product from sample S1 has a size of 904 bp identifying the presence of *Mycobacterium marinum,* PCR product from sample S2 has a size of 831 bp identifying the presence of *Mycobacterium hippocampi,* while PCR product from sample S3 has a size of 538 bp identifying the presence of *Mycobacterium pseudoshottsii.* Lane A is a DNA ladder control with DNA fragments of known size.

### Example 3: Identification of Mycobacterium strains isolated by Argyrosomus regius and/or Dicentrarchus labrax by a Polymerase Chain Reaction (PCR)

For the validation of the selected primers, described in Examples 1 and 2, samples of fish tissues belonging to *Argyrosomus regius* and *Dicentrarchus labrax* species, and showing symptoms of granulomatosis and/or mycobacteriosis were used in a Polymerase Chain Reaction. To this end, whole DNA was isolated from the fish samples following standard genomic DNA extraction and purification techniques. The exracted DNA was then used for specific amplification by a polymerase chain reaction. Moreover, whole DNA extracted by previously isolated *Mycobacterium pseudoshottsii* strain AR, *Mycobacterium hippocampi* strain DL and *Mycobacterium marinum* (as identified by standard microbiological techniques e.g. Medical Microbiology; https://www.ncbi.nlm.nih.qov/books/NBK8406/) were used as controls (see also Figures 1 and 2).

### A: Identification of Mycobacterium pseudoshottsii strain AR and Mycobacterium hippocampi strain DL in fish tissue samples.

For the identification of potential presence of the above mentioned mycobacterial strains in fish tissue samples, a polymerase chain reaction, using the DNA primers of Example 1, was designed. In brief, for the DNA amplification one cycle at 95 ⁰C for 2 min was performed, followed by 35 cycles at 95 ⁰C for 30 seconds (denaturation), at 50 ⁰C for 30 seconds (annealing), at 72 ⁰C for 50 seconds (extending), and one cycle at 72 ⁰C for 2 min. The DNA amplification was performed in 25 µL reaction mixtures containing 2.5 µL KAPA Taq buffer (KAPA BioSystems, Wilmington,USA) 10×, 0.25 µL dNTPs (25 mM), 0.25 µL of KAPA Taq DNA polymerase, 0.5 µL of the forward primer (25 µM), 0.5 µL of the reverse primer (25 µM), 1 µL of template DNA solution, and was finalized with 20 µL sterile deionized water.

The amplified PCR products were then run in an agarose gel electrophoresis in order to identify and the size and thus the origin of the amplified DNA fragment. Figure 3 shows a gel electrophoresis of the amplified DNA products from four tissue samples, with two samples isolated from *Argyrosomus regius* fish tissue (B and D) and two samples isolated from *Dicentrarchus labrax* fish tissue (A and C). The PCR product from sample A, measuring 380 bp, indicates the presence of *Mycobacterium hippocampi.* Sample B's PCR product, measuring 269 bp, identifies the presence of *Mycobacterium pseudoshottsii.* Sample C shows two bands: one at 380 bp, indicating *Mycobacterium hippocampi,* and another at 269 bp, indicating the co-presence of *Mycobacterium pseudoshottsii.* No amplification was observed for sample D, suggesting the absence of both *Mycobacterium pseudoshottsii* strain AR and *Mycobacterium hippocampi* strain DL. Lane O is a DNA ladder control with DNA fragments of known size.

### B: Identification of Mycobacterium pseudoshottsii strain AR, Mycobacterium hippocampi strain DL and Mycobacterium marinum in fish tissue samples

For the identification of potential presence of the above mentioned mycobacterial strains in fish tissue samples a polymerase chain reaction, using the DNA primers of Example 2, was designed. In brief, for the DNA amplification one cycle at 95 ⁰C for 2 min was performed, followed by 35 cycles at 95 ⁰C for 30 seconds (denaturation), at 56 ⁰C for 30 seconds (annealing), at 72 ⁰C for 60 seconds (extending), and one cycle at 72 ⁰C for 2 min. The DNA amplification was performed in 25 µL reaction mixtures containing 2.5 µL KAPA Taq buffer (KAPA BioSystems, Wilmington,USA) 10×, 0.25 µL dNTPs (25 mM), 0.25 µL of KAPA Taq DNA polymerase, 0.5 µL of the forward primer (25 µM), 0.5 µL of the reverse primer (25 µM), 1 µL of template DNA solution, and was finalized with 20 µL sterile deionized water.

The amplified PCR products were then run in an agarose gel electrophoresis in order to identify and the size and thus the origin of the amplified DNA fragment. Figure 1 shows the amplified DNA products from samples isolated by an *Argyrosomus regius* fish (S2) and and *Dicentrarchus labrax* fish (S1). As it can be seen, the PCR product from sample S1 has a size of 380 bp identifying the prsesnce of *Mycobacterium hippocampi,* while PCR product from sample S2 has a size of 269 bp identifying the prsesnce of *Mycobacterium pseudoshottsii.* Figure 4 shows a gel electrophoresis of amplified DNA products from five tissue samples, with two samples from *Argyrosomus regius* fish (C and E) and three from *Dicentrarchus labrax* fish (A, B, and D). The PCR product from sample A, measuring 831 bp, indicates the presence of *Mycobacterium hippocampi.* Sample B's PCR product, at 904 bp, identifies *Mycobacterium marinum.* Sample C's PCR product, sized at 538 bp, reveals the presence of *Mycobacterium pseudoshottsii.* Sample D shows three bands: one at 831 bp, indicating *Mycobacterium hippocampi;* another at 904 bp, indicating the co-presence of *Mycobacterium marinum;* and a third at 538 bp, indicating the co-presence of *Mycobacterium pseudoshottsii.* For sample E, no amplification was observed, suggesting the absence of *Mycobacterium pseudoshottsii* strain AR, *Mycobacterium marinum,* and *Mycobacterium hippocampi* strain DL. Lane O is a DNA ladder control with DNA fragments of known size.

## Claims

1. A pair of DNA primers for detecting and/or distinguishing a *Mycobacterium* species in a sample isolated by a fish or a marine animal wherein
i. the forward primer of said pair of DNA primers comprises or consists of the sequence of SEQ ID NO: 1 and the reverse primer of said pair of DNA primers comprises or consists of the sequence of SEQ ID NO: 2, and wherein said pair of primers is suitable for detecting and/or distinguishing the presence of *Mycobacterium pseudoshottsii* strain AR and/or *Mycobacterium hippocampi* strain DL; or
ii. the forward primer of said pair of DNA primers comprises or consists of the sequence of SEQ ID NO: 3 and the reverse primer of said pair of DNA primers comprises or consists of the sequence of SEQ ID NO: 4, and wherein said pair of primers is suitable for detecting and/ or distinguishing the presence of *Mycobacterium marinum* and/or *Mycobacterium pseudoshottsii* strain AR and/or *Mycobacterium hippocampi* strain DL.

2. The pair of DNA primers of claim 1 for detecting and/or distinguishing *Mycobacterium* species in a sample isolated by a fish or a marine animal in a polymerase chain reaction.

3. The pair of DNA primers of claim 2 for detecting and/ or distinguishing Mycobacterium species in a sample isolated by a fish or a marine animal, wherein
i. after completion of said polymerase chain reaction using forward primer having the sequence of SEQ ID NO: 1 and reverse primer having the sequence of SEQ ID NO: 2, the production of a DNA fragment having a size of 380 bp indicates the presence of *Mycobacterium hippocampi,* and/or the production of a DNA fragment having a size of 269 bp indicates the presence of *Mycobacterium pseudoshottsii;* or
ii. after completion of said polymerase chain reaction using forward primer having the sequence of SEQ ID NO: 3 and reverse primer having the sequence of SEQ ID NO: 4, the production of a DNA fragment having a size of 904 bp indicates the presence of *Mycobacterium marinum,* and/or the production of a DNA fragment having a size of 831 bp indicates the presence of *Mycobacterium hippocampi,* and/or the production of a DNA fragment having a size of 538 bp indicates the presence of *Mycobacterium pseudoshottsii.*

4. A composition comprising a DNA primer pair selected from the group consisting of SEQ ID NOs: 1 and 2 and SEQ ID NOs: 3 and 4.

5. The composition of claim 4, wherein said composition is a reaction mixture.

6. The composition of claim 5, wherein said reaction mixture comprises a sample.

7. The composition of claim 6, wherein said sample comprises a *Mycobacterium marinum* or *Mycobacterium hippocampi* or *Mycobacterium pseudoshottsii* target nucleic acid suitably DNA.

8. A method of detecting and/ or distinguishing the presence of a *Mycobacterium* strain in a sample isolated by a fish or marine animal, **characterised in that** a pair of DNA primers according to any one of claims 1 to 3 is used in order to amplify a substrate DNA comprised in said sample.

9. The method of claim 8, wherein said method is a polymerase chain reaction.

10. The method of claim 9, wherein
i. after completion of said polymerase chain reaction using forward primer having the sequence of SEQ ID NO: 1 and reverse primer having the sequence of SEQ ID NO: 2, the production of a DNA fragment having a size of 380 bp indicates the presence of *Mycobacterium hippocampi,* and/or the production of a DNA fragment having a size of 269 bp indicates the presence of *Mycobacterium pseudoshottsii;* or
ii. after completion of said polymerase chain reaction using forward primer having the sequence of SEQ ID NO: 3 and reverse primer having the sequence of SEQ ID NO: 4, the production of a DNA fragment having a size of 904 bp indicates the presence of *Mycobacterium marinum,* and/or the production of a DNA fragment having a size of 831 bp indicates the presence of *Mycobacterium hippocampi,* and/or the production of a DNA fragment having a size of 538 bp indicates the presence of *Mycobacterium pseudoshottsii.*

11. A kit for detecting or distinguishing the presence of a *Mycobacterium* strain in a sample isolated by a fish or marine animal, wherein said kit comprises a pair of DNA primers according to any one of claims 1 to 3 or a composition according to claims 4 to 7.
